# EUROPEAN PATENT SPECIFICATION

(11) **EP 2 173 248 B1**
(45) Date of publication and mention of the grant of the patent: **05.11.2014**
(21) Application number: 08838965.5
(22) Date of filing: 16.04.2008
(51) Int. Cl.: A61B 5/055, A61B 19/00, B25J 19/04, G01R 33/48

(54) **METHODS, DEVICES, AND SYSTEMS RELATING TO CAMERAS CONFIGURED TO BE POSITIONED WITHIN THE BORE OF A MAGNET AND MR BORE SPACE ILLUMINATION**
VERFAHREN, VORRICHTUNGEN UND SYSTEME IN ZUSAMMENHANG MIT KAMERAS ZUR POSITIONIERUNG IN DER BOHRUNG EINES MAGNETEN UND MR-BOHRUNGSRAUMBELEUCHTUNG
PROCÉDÉS, DISPOSITIFS, ET SYSTÈMES CONCERNANT DES CAMÉRAS CONFIGURÉES POUR ÊTRE POSITIONNÉES DANS L'ALÉSAGE D'UN AIMANT ET ÉCLAIRAGE DE L'ESPACE D'ALÉSAGE RM

(30) Priority: 16.04.2007 US 912148 P
(43) Date of publication of application: 14.04.2010
(73) Proprietor: NeuroArm Surgical, Ltd., Calgary, AB T2N 2T9 (CA)
(72) Inventor: GREER, Alexander, Calgary, Alberta T2N 1N4 (CA); SUTHERLAND, Garnette, Calgary, Alberta T2N 1N4 (CA); FIELDING, Tim, Calgary, Alberta T2N 1N4 (CA); NEWHOOK, Perry, Calgary, Alberta T2N 1N4 (CA); YU, Simon, Calgary, Alberta T2N 1N4 (CA)
(74) Representative: Finnie, Peter John
(86) International application number: PCT/IB2008/003669
(87) International publication number: WO 2009/050589

(56) References cited:
- EP-A2- 1 559 363
- WO-A1-03/054568
- JP-A- 2001 057 968
- US-A1- 2005 054 910
- US-B2- 7 155 316
- US-B2- 7 155 316
- NEUNER ET AL.: 'Development and implementation of an MR-compatible whole body video system' NEUROSCIENCE LETTERS vol. 420, 06 April 2007, pages 122 - 127, XP022098654

## Description

### BACKGROUND INFORMATION

The present methods, devices, and systems relate generally to the field of surgical robotics, and more particularly to cameras systems that can be used during surgical procedures involving a surgical robot and to techniques for illuminating and capturing images of space within an open or closed bore of a magnetic resonance imaging (MRI) system. An example of a surgical robot that can be used in a procedure to which the present methods, devices, and systems relate is disclosed in U.S. Patent No. 7,155,316 (the '316 patent), which discloses a camera system according to the preamble of the accompanying claim 1.

### SUMMARY

Broadly, the present invention concerns methods and systems for illuminating and/or obtaining images of operating space within an MRI system, including when the MRI system is taking magnetic resonance (MR) images of an object, such as a patient. For example, embodiments of the present camera systems may be used during stereotactic procedures.

The invention is defined in claim 1 and claim 11. Further embodiments are defined in the respective dependent claims.

Some embodiments of the present camera systems are for use with a manipulator configured to be secured to an extension member (e.g., an extension board) that can be coupled to an operating table on which a patient can be positioned for a stereotactic procedure. The manipulator may be operable to move a surgical instrument (or tool) located within the bore of a magnet of an MRI system. Such camera systems may comprise a first magnetic resonance (MR) compatible camera system configured to be coupled to the extension member; and a second MR-compatible camera system configured to be coupled to the extension member.

Some embodiments of the present methods comprise illuminating a space within the bore of a magnet of a magnetic resonance imaging (MRI) system with a light emitting diode (LED) light source coupled to a camera positioned within the bore, the illuminating occurring during a surgical procedure on a patient located at least partially within the bore.

Some embodiments of the present methods may comprise illuminating a first portion of a patient positioned at least partially within a bore of a magnet of a magnetic resonance imaging (MRI) system with a first light emitting diode (LED) light source coupled to a first camera positioned within the bore; and illuminating a second portion of the patient with a second LED light source coupled to a second camera positioned within the bore; where the first and second portions overlap to at least some extent, and the illuminating of both portions occurs at the same time and during a surgical procedure on the patient.

Some embodiments of the present methods may comprise positioning a first LED light source coupled to a first camera in a bore of a magnet of a magnetic resonance imaging (MRI) system; and positioning a second LED light source coupled to a second camera in the bore.

Some embodiments of the present methods may comprise orienting two of the present camera systems at a distance apart from each other and at an angle toward a site to be imaged such that they capture views that can be presented to a user as a three-dimensional stereoscopic image (that, e.g., provides perspective and depth) through a suitable display device, such as a microscope viewer, a microscope binocular tube, stereoscope eyepieces, or a stereoscopic display unit. Such methods may also comprise outputting the 3D stereoscopic image for viewing by a user.

Some embodiments of the present methods may comprise positioning an LED light source coupled to a camera in a bore of a magnet of a magnetic resonance imaging (MRI) system; and supplying power to both the LED light source and the camera while the MRI system images an object.

Any embodiment of any of the present methods, devices, and systems may consist of or consist essentially of-rather than comprise/include/contain/have-the described steps and/or features. Thus, in any of the claims, the term "consisting of" or "consisting essentially of" may be substituted for any of the open-ended linking verbs recited above, in order to change the scope of a given claim from what it would otherwise be using the open-ended linking verb.

### BRIEF DESCRIPTION OF THE FIGURES

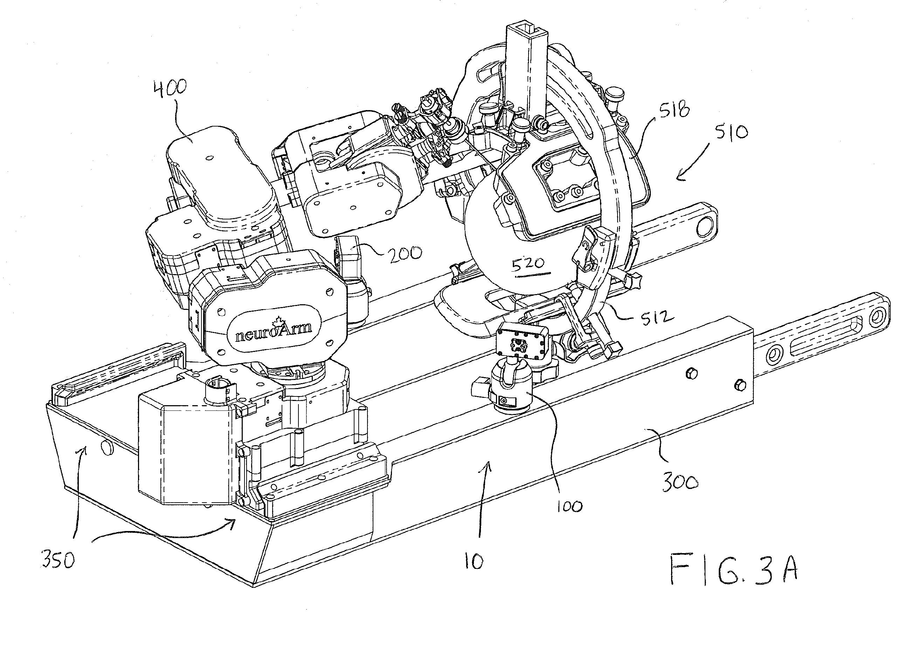

The following drawings illustrate by way of example and not limitation. Identical reference numerals do not necessarily indicate an identical structure. Rather, the same reference numeral may be used to indicate a similar feature or a feature with similar functionality. Every feature of each embodiment is not always labeled in every figure in which that embodiment appears, in order to keep the figures clear. The camera system structures shown in the figures (as well as the manipulator and the assembly shown in FIG. 3A) are drawn to scale, meaning the sizes of the depicted elements are accurate relative to each other.
FIG. 1 is a perspective view of one embodiment of the present imaging systems comprising two camera systems mounted on an extension board;
FIG. 2 is a top view of the imaging system embodiment of FIG. 1;
FIG. 3A is a perspective view of the imaging system embodiment of FIG. 1 in an environment where a manipulator is mounted on the extension board;
FIGS. 3B and 3C are views that may be taken with an embodiment of a two-camera system version of the present imaging systems.
FIGS. 4A and 4B are perspective views (FIG. 4B being an enlarged view of a portion of what is shown in FIG. 4A) of the imaging system embodiment of FIG. 1 used in an open bore MRI system;
FIG. 5 is a perspective view of the imaging system embodiment of FIG. 1 used in a closed bore MRI system;
FIG. 6 is an end view of the arrangement shown in FIG. 5;
FIG. 7 is a front perspective view of a camera system of the imaging system embodiment of FIG. 1;
FIG. 8A depicts components of a camera that may be used with embodiments of the present camera systems;
FIG. 8B depicts components of a light source (specifically, an LED light source) that may be used with embodiments of the present camera systems;
FIG. 9A is a back view of the camera system of FIG. 7;
FIG. 9B is a perspective view of the camera system of Fig. 7 that includes a camera (not visible) and an LED light source in the camera system casing, as well as a conduit channeling cables to both the camera and LED light source; and
FIGS. 10 and 11A-11C illustrate a schematic view of an exemplary embodiment of a wiring system for the camera system shown in FIG. 7.

### DETAILED DESCRIPTION OF ILLUSTRATIVE EMBODIMENTS

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. Thus, a method comprising certain steps is a method that includes at least the recited steps, but is not limited to possessing only the recited steps. Similarly, a camera system comprising certain elements or features includes at least those recited, but is not limited to possessing only the recited elements/features. Furthermore, a stricture that is configured in a certain way must be configured in at least that way, but also may be configured in a way or ways that are not specified.

The terms "a" and "an" are defined as one or more than one, unless this application expressly requires otherwise. The term "another" is defined as at least a second or more. The terms "substantially" is defined as at least close to (and includes) a given value or state (preferably within 10% of, more preferably within 1% of, and most preferably within 0.1% of).

The inventors have designed camera systems that may be used safely in the bore of an open or closed magnet of an MRI system (also known as open or closed MRI systems). They were guided by trying to ensure safe operation of the camera system while inside the MRI system, as well as safe operation of the MRI system while the camera system is inside the MRI system. They were also guided by trying to ensure that the image(s) their camera systems produce is not significantly degraded by operation of the MR magnet when a scan is taken as the camera system is taking images, nor is the image(s) significantly degraded by the camera system's presence or operation. The camera system and MRI system should be compatible so as to avoid causing unwanted movement of material, heat generation, or electrical transmissions due to magnetic forces.

The inventors' efforts to meet their goals resulted in the design of camera systems having a minimized amount of magnetic material in the camera and in the light source for illuminating the space imaged by the camera, as well as an ability to function in strong magnetic fields, such as 1.5 or 3.0 Tesla superconducting magnets. The inventors took steps to minimize and shield the radio frequency (RF) noise created by the camera and light of their camera systems, so that the noise stays inside of a sealed environment (for example, the housing for the camera and the light and the cable or cables supplying power and/or receiving data from the light and camera). The inventors note that it can also be beneficial to transmit RF noise to a ground outside of the magnetic resonance (MR) environment. Examples are provided below of filtering and shielding techniques, which will vary from implementation to implementation depending on the magnet style, that may be used in combination with the present camera systems when embodiments of the present camera systems are used for taking images during MRI scans.

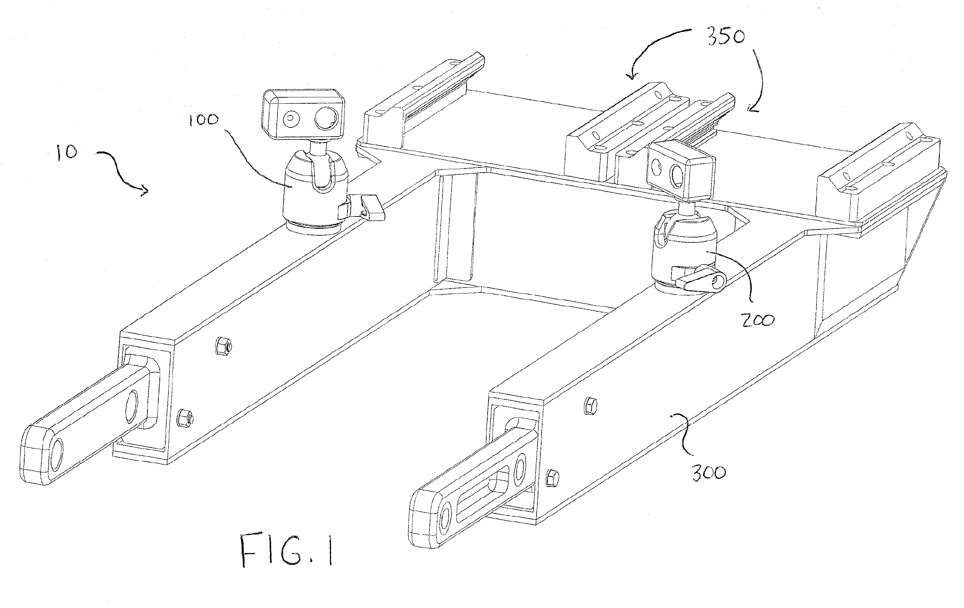

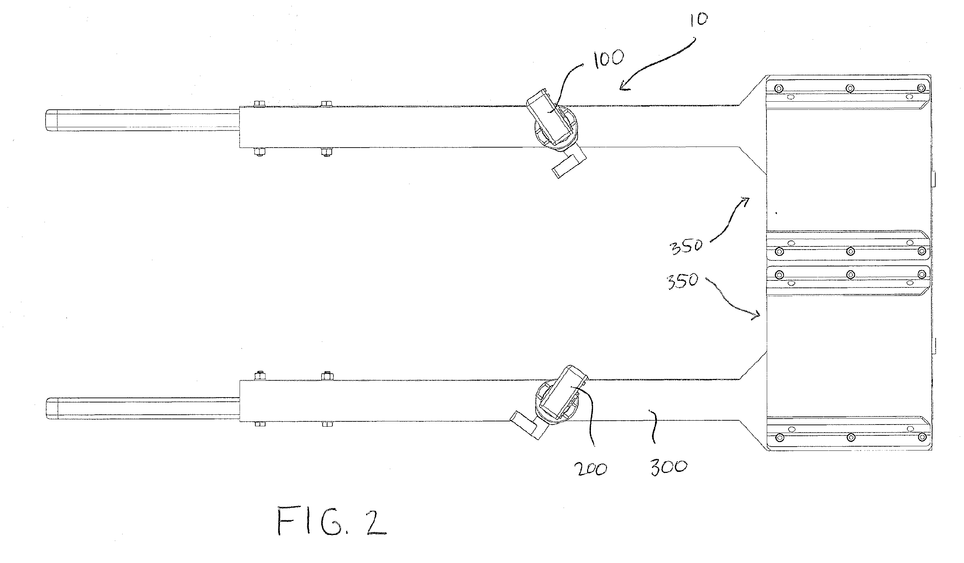

Referring initially to FIGS. 1 and 2, perspective and top views are shown of one of the present imaging systems: imaging system 10, which comprises a right camera system 100 and a left camera system 200, both of which are examples of the present camera systems and both of which are mounted on an extension board 300 (not part of the imaging system in some embodiments) that can be coupled to a patient support surface (not shown in FIGS. 1 and 2). (The present imaging systems may also be referred to as camera systems that include a first camera system and a second camera system.) Extension board 300 comprises a pair of docking stations 350 that each comprises a pair of mounting tracks for a robotic arm (also termed a (slave) manipulator). As shown in FIG. 3A, each docking station 350 can support a manipulator 400 (including, for example, the manipulators of the surgical robot disclosed in the 316 patent). FIG. 3A also shows a graphical representation of a subject's (e.g., a patient's) head 520 that is supported by an assembly 510 that includes a head clamp 512 to which a radio frequency (RF) coil device 518 is coupled, and shows that the left and right camera systems 200 and 100 may be positioned between manipulator 400 and the patient's head 520.

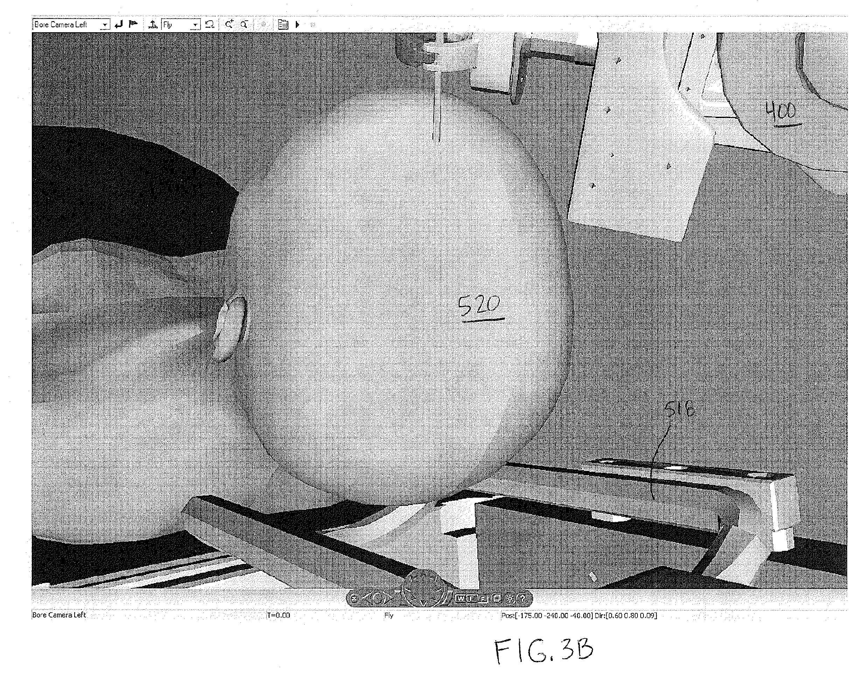

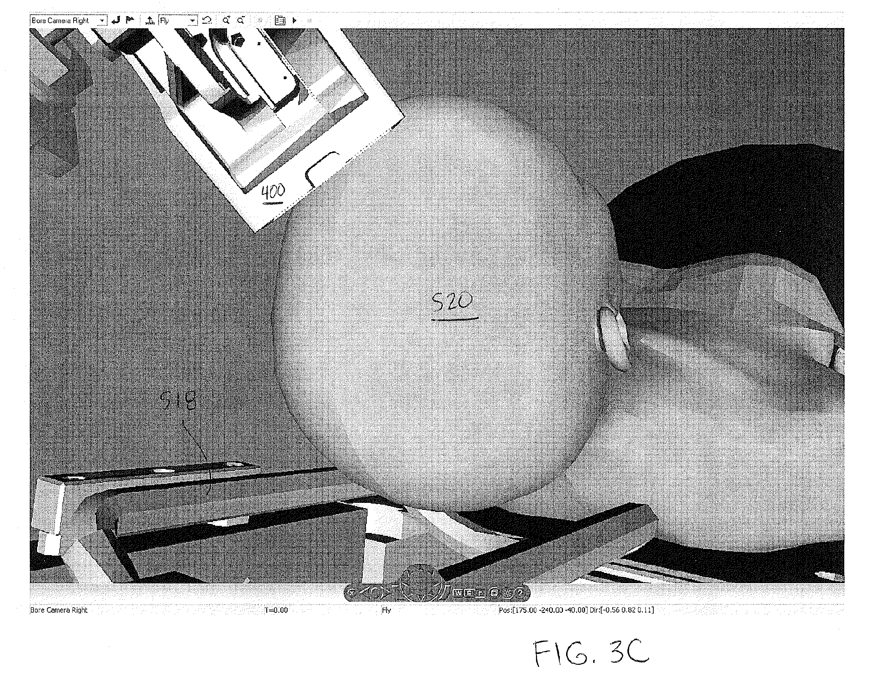

The camera systems can be oriented (as discussed in more detail below) such that the light source of each illuminates a different area of the operating space, and so that the camera of each captures an image of a different area of the operating space. However, the areas illuminated may overlap as may the subject matter captured in the image(s) obtained by each camera. FIG. 3B shows a graphical representation of an example of a view that can be obtained by left camera system 200 of an operating space (which can be characterized as a "left bore view"), and FIG. 3C shows a graphical representation of an example of a view that can be obtained by right camera system 100 of the operating space (which can be characterized as a "right bore view"). These figures illustrate the potential for overlapping illumination and image capture of the operating space of a surgical procedure. Graphical representations of an alternative version of manipulator 400, and an alternative version of a portion of RF device 518 are shown in FIGS. 3B and 3C.

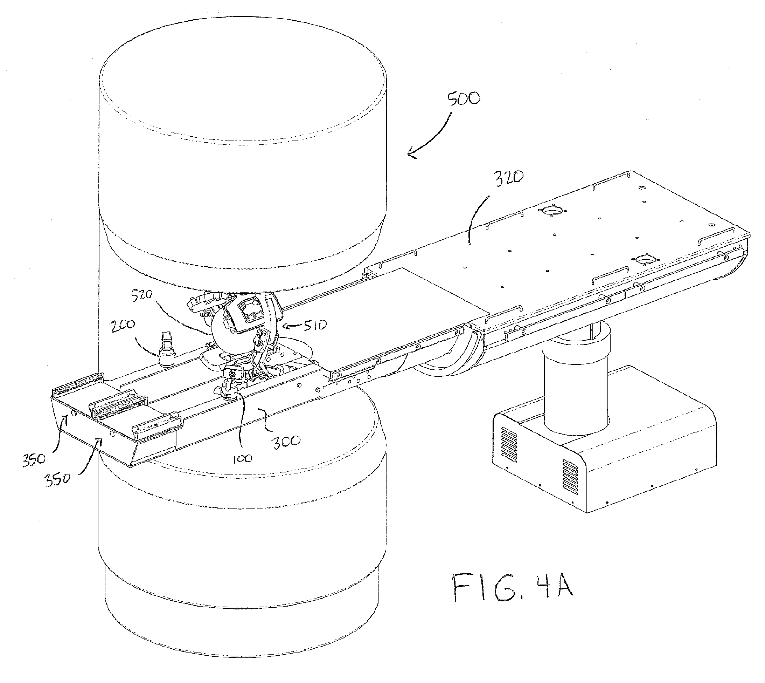

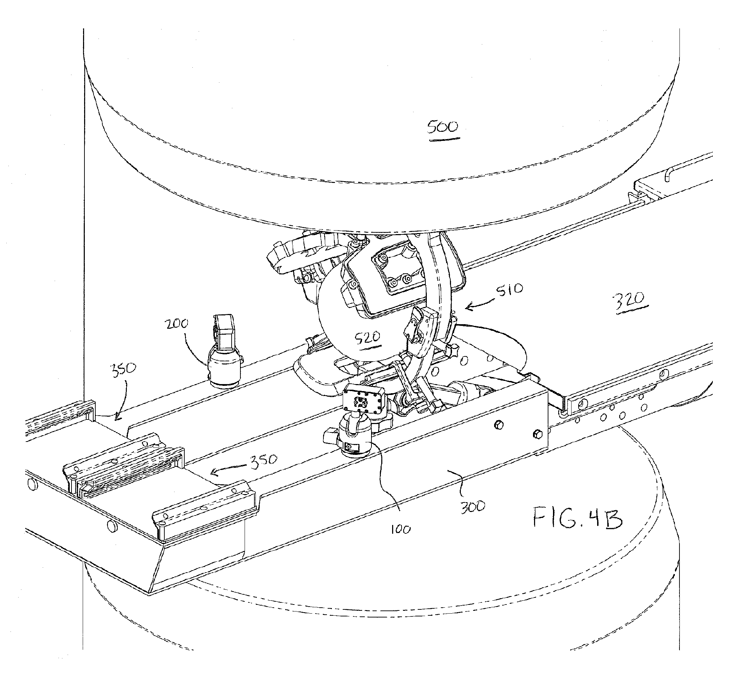

FIGS. 4A and 4B show that imaging system 10 may be used in an open bore MRI system 500. In the embodiment shown in these figures, extension board 300 is coupled to patient support surface 320, which is the top surface of an operating table. As shown, right and left camera systems 100 and 200 can be positioned such that they are bounded by (or within the vertically-oriented cylindrical perimeter defined by) the open bore magnet of system 500, and are directed toward head 520 of the subject to be operated on.

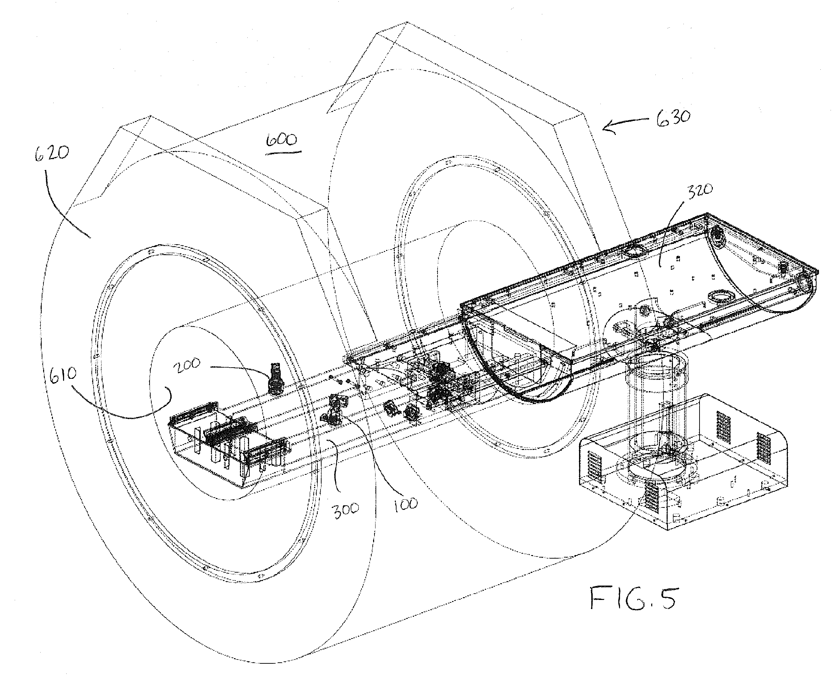

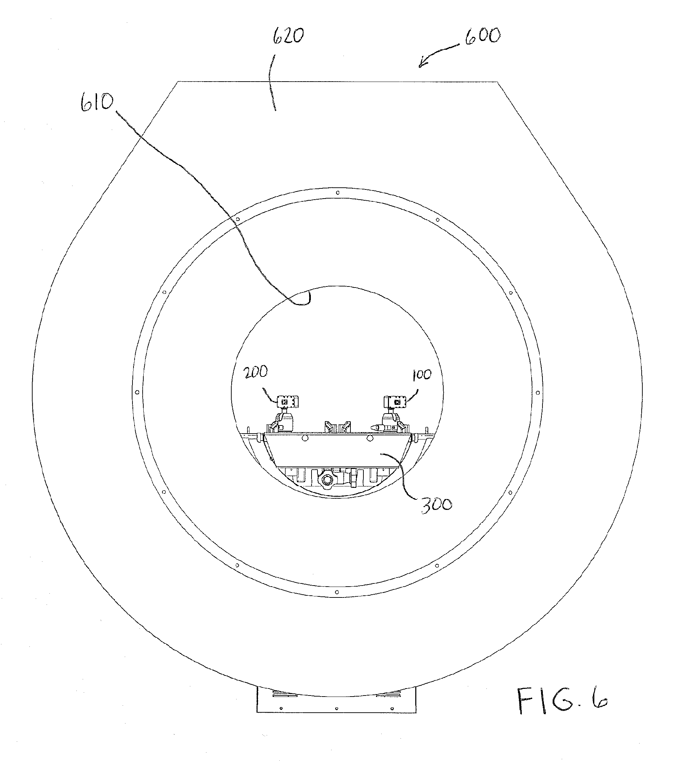

In FIG. 5, a perspective view of a closed bore MRI system 600 is shown in phantom so that the components placed within bore 610 of closed bore MRI system 600 are visible. System 600 may be a closed bore 1.5 Tesla MRI system. In this configuration extension board 300, as well as right and left camera systems 100 and 200, are shown within bore 610. Extension board 300 can be inserted into end 630 of bore 610 such that the docking stations are positioned proximate to end 620 of system 600. As shown, right and left camera systems 100 and 200 can be positioned such that they are bounded by (or within the laterally-oriented cylindrical perimeter defined by) the closed bore magnet of system 600, and can directed toward the head of a subject to be operated on. Referring now to FIG. 6, a view into bore 610 (as viewed from end 620) shows that right and left camera systems 100 and 200 are positioned to look toward a central region within bore 610.

As shown in FIGS. 4A-6, right and left camera systems 100 and 200 of imaging system 10 may be positioned to provide images of a subject area in either a closed bore or an open bore MRI configuration. In exemplary embodiments, camera systems 100 and 200 can be positioned such that, together, their collective field of view encompasses the usable working area of one or more manipulators used during a procedure involving the use of an MRI system.

In preferred embodiments, camera systems 100 and 200 are MR-compatible. This means, generally, that the camera system is MR-safe (being in the MRI system does not present a hazard to either the equipment or the subject); operation of the camera system is not adversely affected by operation of the MRI machine (and, more broadly, the MRI system of which the MRI machine is a part); operation of the MRI machine (and, more broadly, the MRI system of which the MRI machine is a part) is not adversely affected by operation of the camera system; and the MR image is not significantly affected by the presence of the camera system such that a maximum decrease in signal to noise ratio (SNR) of ten percent is permissible compared to when no camera system is present. In addition, the presence of one or both camera systems 100 and 200 should create minimal distortion of the images produced by the MRI system. SNR is calculated as: mean value of the signal divided by the standard deviation of the noise. Image distortion can happen when the local magnetic field is distorted by the presence of a magnetic or conductive material. Image distortion can take the form of signal dropout, such as where a viewer sees a black area, a lack of signal, or a geometrically distorted object.

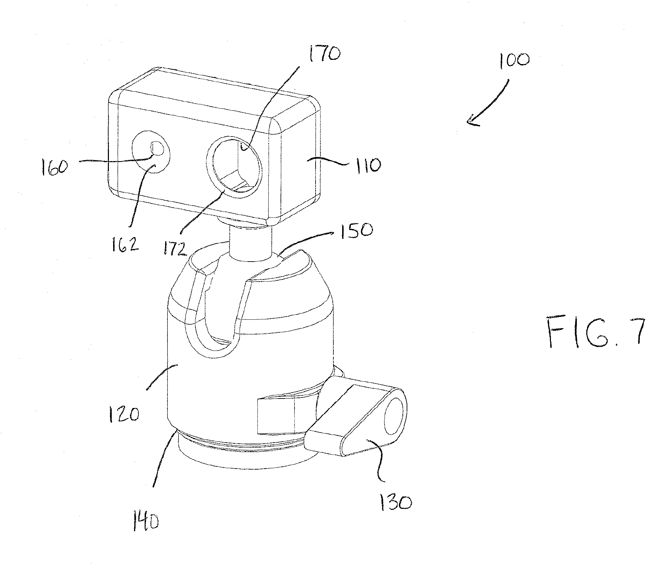

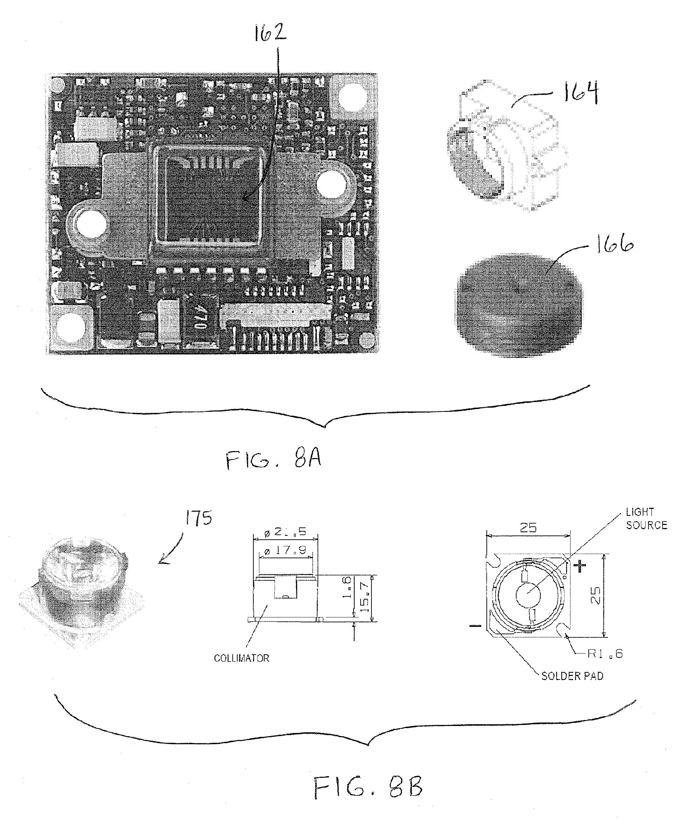

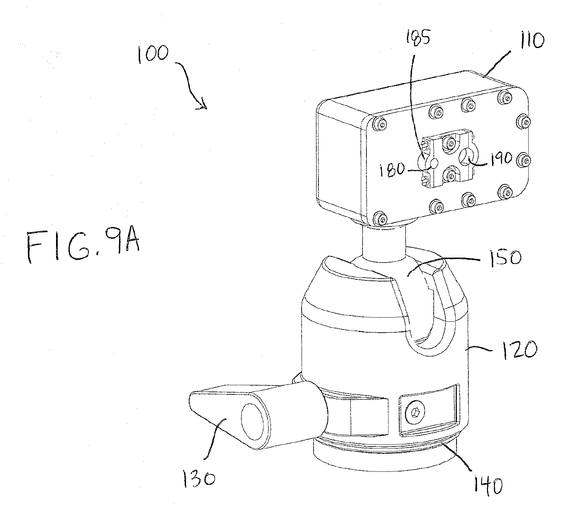

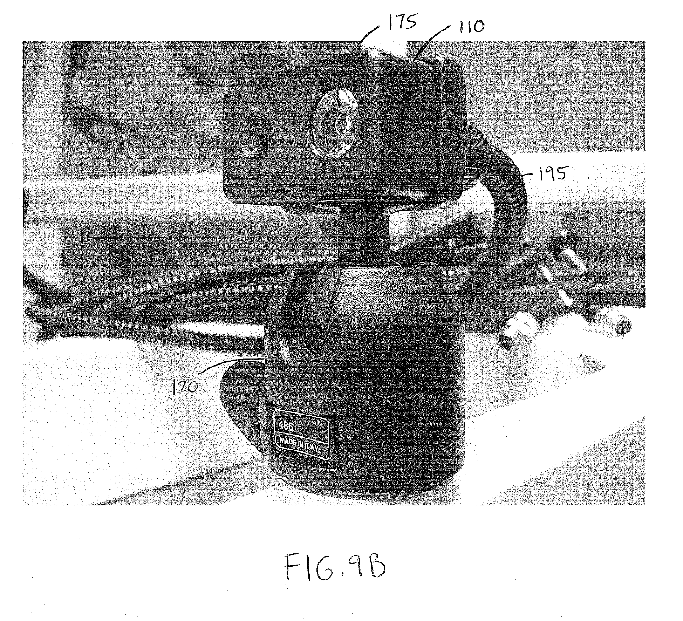

Referring now to FIGS. 7-9B, more detailed views are presented showing one embodiment of the present camera systems. Camera system 100 (note that the designations left and right in the foregoing description and figures do not signify functional or structural differences between the depicted camera systems; the two are substantially identical to each other in some embodiments, and identical in some embodiments) includes a head or upper portion 110 coupled to a body or base 120 via an adjustment mechanism 150 (for example, a swivel mount). Upper portion 110 comprises two openings, including a camera opening 160 and a light opening 170. FIG. 8A shows an example of a camera, which includes an actual camera 162 (attached to a board (unlabeled)), mount 164 to which the board on which camera 162 is attached may be mounted, and a pin-hole lens 166 (which fits inside of the mount and behind which the camera is positioned), that may be enclosed within upper portion 110 and oriented so that the lens is aligned with opening 160. FIG. 8B shows a light source generally designated as 175, that may include a collimator and a housing (both shown but unlabeled), that is contained within upper portion 110 and directed so that at least some light emitted from the light source passes through light opening 170. As shown in the back view of camera system 100 in FIG. 9A, upper portion 110 may also comprise an opening 180 for wiring to light source 175 and an opening 190 for wiring to the camera (e.g., the one shown in FIG. 8A) contained within upper portion 110. Such wiring may be contained within conduit 195 shown in FIG. 9B that houses the two cables (shown in the back of the figure) and is connected to connection adapter 185 of upper portion 110. FIG. 9B also shows light source 175 contained within upper portion 110 (as is a camera, though it is not visible at the depicted angle).

In specific embodiments, upper portion 110 may be a casing comprised of aluminum (or another suitable non-magnetic material) with separate cavities (or a single cavity that includes sufficient structural separations to prevent any radio frequency noise from the camera from entering the portion of the cavity occupied by the light source) for a camera and light source 175. In certain embodiments, upper portion 110 may act as a heat sink to help dissipate any heat generated by the camera and the light source. Camera opening 160 may be offset from the forwardmost surface of upper portion 110 via a tapered section 162 and may be smaller than light opening 170 (which also may be offset from the forwardmost surface of upper portion 110 via a tapered section 172, which is not as deep as tapered section 162), to reduce the amount of RF noise released by camera system 100 into the imaging environment. In certain embodiments, the DC power to light source 175 may be filtered, unlike the signal from the camera. The smaller camera opening 160 is, the more reduction can be achieved in the RF noise released to the imaging environment created by operation of the camera. The larger light opening 170 is, the more light can be provided to the subject area.

In specific embodiments, the camera shown in FIG. 7A may be a small (e.g., 1/4 inch) charge-coupled device (CCD) color board camera that contains no critical magnetic or iron-core components (and minimal non-critical magnetic components, which can be replaced with non-magnetic components). The camera is therefore not attracted to a magnet, including those used in open and closed bore MRI systems. In still more specific embodiments, the camera may be one chosen from the Videology® 20K15X series of cameras (available from Videology Imaging Solutions, Inc., Greenville, Rhode Island), and may in particular be 20K152 with any compatible Flat M-12, Cone M-12, or Semi-Cone M-12 mount and pinhole lens combination (including the combination shown in FIG. 8A, the lens of which is part number 33S5545N, which has a focal length of 5.5 millimeters and a minimum object distance of 50 centimeters).

In certain embodiments, light source 175 may be selected to provide adequate brightness in confined spaces, including closed bore MRI systems. In specific exemplary embodiments, light source 175 may be a white light-emitting diode (LED). In still more specific exemplary embodiments, light source 175 may be a Luxeon® LXHL-NWE8 light, which may be obtained from Phillips Lumileds Lighting Company (San Jose, California) and is described on Lumileds Technical Datasheet DS23. Utilizing a light source that requires only DC power will minimize RF interference (RFI) associated with using the light source, but it may be desirable to filter the DC power source to minimize the chance that the cable carrying the DC power acts as an antenna bringing in outside environmental noise into the imaging environment through light source opening 170.

In certain embodiments, opening 180 (for wiring to light source 175) receives wiring such as a 22 American wire gauge (AWG) shielded pair (e.g., Belden 83319, available from any authorized Belden Inc. distributor (a sales office for Belden is in Santa Fe Springs, California)), transmitting direct current (DC) to light source 175. In specific embodiments, opening 190 (for wiring to camera 165) receives wiring such as 22 AWG hookup wire (e.g., Alpha 696-1362, available from any authorized Alpha Wire Company distributor (Alpha Wire Company has an office in Elizabeth, New Jersey). In some embodiments, video signal from camera 165 may be transmitted over a 75 Ohm mini coax cable (e.g., Belden 8218), and the overbraid for the camera power and video may be tinned copper (e.g., Dearborn 92171 from Dearborn Wire and Cable, a Belden Company). Appropriate standard connectors can be configured for use in camera system 100 by replacing magnetic or iron-core components (for example, screws, clamps, or brackets) with non-magnetic components (such as brass equivalents that are off-the-shelf or hand-made, as necessary). Connectors that may be used with the present systems include those having original manufacturer part numbers: Amphenol T3504 001, and Amphenol T3300 001 (both are available from any authorized Amphenol distributor).

In certain embodiments, upper portion 110 comprises an aluminum enclosure that acts as an RF shield, so that any RFI coming from DC power to the camera, and from the camera itself, gets conducted along the enclosure, and back out along the cable to the system's shield. Upper portion 110 and the shielded cable coming from opening 190 can act as a bubble where RFI is allowed on the inside, but does not reach the imaging environment.

In the embodiment shown, base 120 comprises a coupler 140 (for example, a threaded portion) that allows camera system 100 to be coupled to extension board 300. In one specific embodiment, camera system 100 is coupled to extension board 300 approximately 40-75 centimeters (or any distance between) from the subject area being viewed (for example, a patient's head). A handle or controller 130 is coupled to base 120 and allows adjustment mechanism 150 to be placed in a locked or an unlocked position. In the locked position, adjustment mechanism 150 is restrained from moving so that upper portion 110 is fixed relative to base 120. In the unlocked position, adjustment mechanism 150 can be adjusted to that upper portion 110 is allowed to move relative to base 120. In the unlocked position, a user can adjust upper portion 110 so that camera system 100 is directed toward a desired field of view. When so positioned, light source 175 and the camera are both aimed at the desired subject area. After upper portion 110 is in the desired position, a user may place controller 130 in the locked position to retain upper portion 110 in place.

With camera systems 100 and 200 properly positioned, a user may conduct a procedure in which MRI imaging is performed while camera systems 100 and 200 transmit images of their respective portions of the subject viewing area. The images provided by camera systems 100 and 200 can provide useful information to the user that can assist in successfully performing and analyzing the procedure.

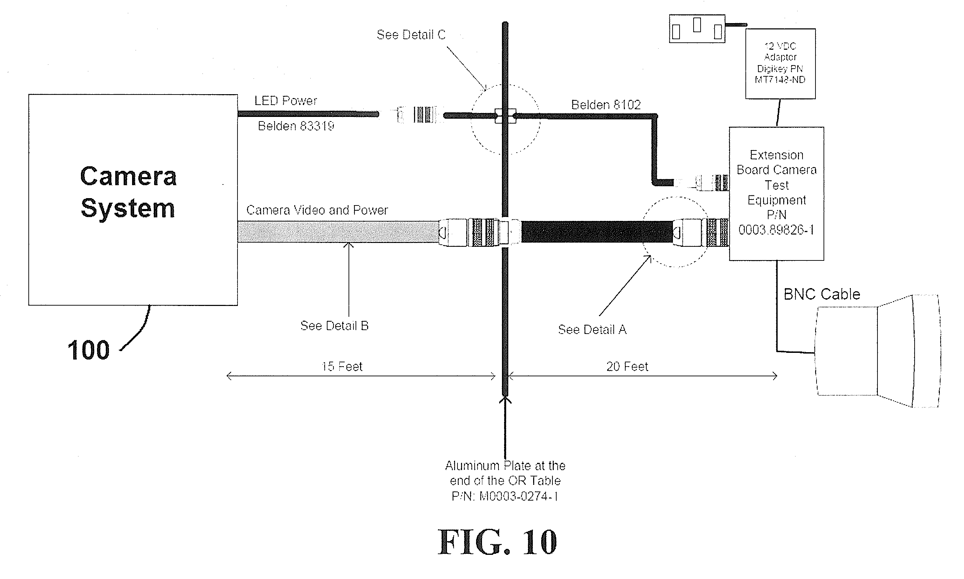

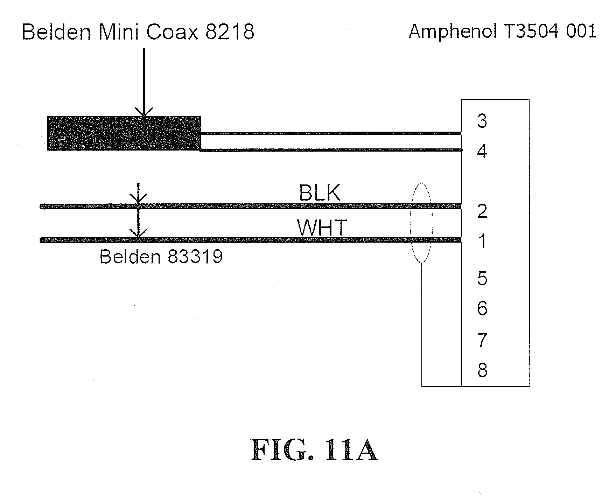

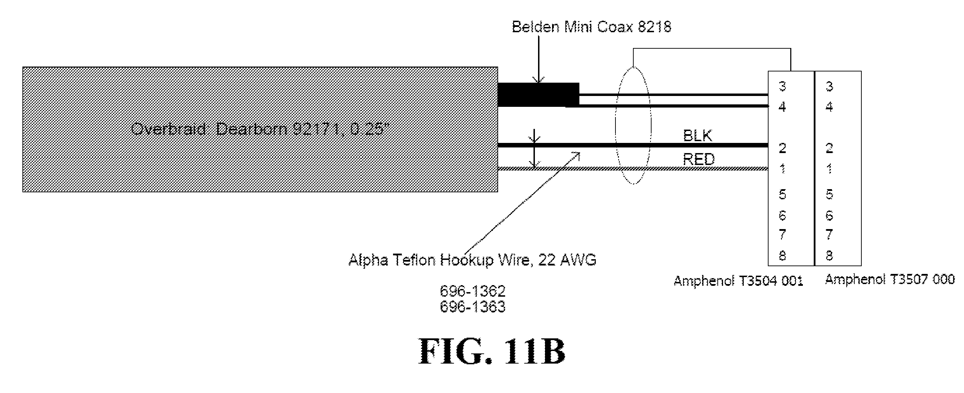

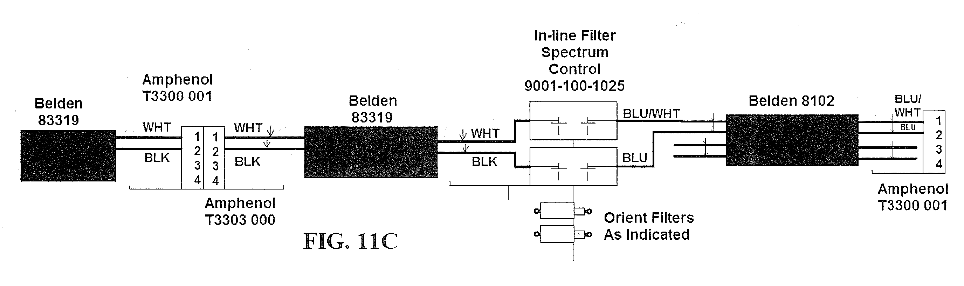

FIGS. 10 and 11A-11C illustrate a schematic view of an exemplary embodiment of a wiring system for camera system 100. FIG. 11A provides a detailed view of the section labeled "Detail A" in FIG. 10. In addition, FIGS. 11B and 11C provide detailed views of the sections labeled "Detail B" and "Detail C" in FIG. 10.

### Shielding Examples

Depending on the desired application for the present imaging systems (which may include one or more of the present camera systems), local shielding or room shielding may be appropriate. For example, using a closed bore 1.5 Tesla ("1.5T") MRI system may involve local shielding while using a closed bore 3.0 Tesla ("3T") MRI system may involve room shielding. A locally shielded magnet may rely on the imaging environment being sealed from RFI through the use of a Faraday cage. For a closed bore magnet, the head-end of the bore may be sealed using a copper-mesh impregnated PLEXIGLAS disk that is attached to the magnet face and shield. The foot end of the bore may be sealed using a Faraday cage that goes over the patient's legs (and sits on the operating room bed) and attaches to the magnet using a silver impregnated mesh. The cage itself may be made of copper wire impregnated PLEXIGLAS. This "dog house" may create an RFI-free environment all around the patient's body, and also help keep powerful RF impulses from being thrown out into the hospital environment. A penetration panel with specialized filters may be used to get cables into and out of the imaging environment without introducing RFI. If the penetration panel is not mounted directly on the Faraday cage, any cables going into the cage should be shielded, and should carry RFI along the cable out of the environment and out to the shield. Embodiments of the present camera systems may be shielded by shielding the housing (as described above) and shield any cables coming into/out of the housing (as described above) such that any RFI gets transmitted to the penetration panel located outside of the imaging environment.

In a room-shielded environment, copper sheets in the walls/floor/ceiling of the operating room create a shielded "box" around the operating room (OR), and the only cables coming into or out of the box do so through penetration panels or wave guides. Penetration panels are specialized filters that filter noise off of cables that act as antennae for RFI (typical copper cables). For cables that do not pick up and carry noise (such as fiber optic cables, plastic air tubes, etc.) wave guides are used. Wave guides are cylindrical penetrations in the wall of the operating room, and provide a minimum length/diameter ratio such that RF waves will not make it through them. With the present camera systems, if the cables for both the light source and the camera are copper, a penetration panel can be used. As in a locally shielded environment, the camera and cable that are inside the MRI environment - here, the whole OR - should create a bubble of RFI that is contained within their shielding, and which sends the RFI back to the penetration panel and room shield.

MR-compatible embodiments of the present camera systems may be configured so as to be useable with either or both local and room shielded magnets that are either the open or closed bore type.

Descriptions of well known techniques, components and equipment have been omitted so as not to unnecessarily obscure the present camera systems and illumination methods in unnecessary detail. The descriptions of the present methods, devices and systems are exemplary and non-limiting. Certain substitutions, modifications, additions and/or rearrangements but not explicitly listed in this disclosure, may become apparent to those of ordinary skill in the art based on this disclosure. Furthermore, it will be appreciated that in the development of a working embodiment, numerous implementation-specific decisions must be made to achieve the developers' specific goals, such as compliance with system-related and business-related constraints, which will vary from one implementation to another. While such a development effort might be complex and time-consuming, it would nonetheless be a routine undertaking for those of ordinary skill in the art having the benefit of this disclosure.

## Claims

1. A camera system (100;200) comprising:
a first magnetic resonance (MR) compatible casing having at least two separate openings (160,170);
a first camera (162) oriented to view a site through one of the separate openings (160); and
a first light emitting diode (LED) light source (175);
**characterised in that** the first LED light source (175) is oriented to illuminate at least a portion of the site through another of the separate openings (170).

2. The camera system of claim 1, further comprising:
a first shielded cable that delivers power to the first LED light source (175); and
first shielded video and power cables coupled to the first camera (162).

3. The camera system of claim 1 or 2, wherein the camera system includes a first upper portion (110) that includes the first camera and the first LED light source, and a first base (120) to which the first upper portion (110) is swivel-mounted.

4. The camera system of claim 3, wherein the orientation of the first upper portion (110) can be adjusted manually after unlocking a controller (130) coupled to the first base (120).

5. The camera system of any one of claims 1 to 4, further comprising:
a second MR-compatible casing having at least two separate openings (160,170);
a second camera (162) oriented to view a second site through one of the separate openings (160) of the second casing; and
a second light emitting diode light source (175) oriented to illuminate at least a portion of the second site through another of the separate openings (170) of the second casing.

6. The camera system of claim 5, further comprising:
a second shielded cable that delivers power to the second LED light source (175); and
second shielded video and power cables coupled to the second camera (162).

7. The camera system of claim 5 or 6, wherein the camera system includes a second upper portion (110) that includes the second camera (162) and the second LED light source (175), and a second base (120) to which the second upper portion (110) is swivel-mounted.

8. The camera system of claim 7, wherein the orientation of the second upper portion (110) can be adjusted manually after unlocking a controller (130) coupled to the second base (120).

9. The camera system of any one of claims 5 to 8, for use with a manipulator (400) configured to be secured to an extension member (300) that can be coupled to an operating table (320) on which a patient can be positioned for a stereotactic procedure, the manipulator (400) being operable to move a surgical instrument located within the bore (610) of an MRI magnet (500;600), wherein the first and second camera systems (100,200) are each configured to be coupled to the extension member (300).

10. The camera system of claim 9, wherein the first and second camera systems (100,200) each includes a pinhole lens (166).

11. A method comprising:
illuminating a first portion of a patient located at least partially within the bore (610) of a magnet of a magnetic resonance imaging (MRI) system (500;600) with a first light emitting diode (LED) light source (175) coupled to a first camera (162) positioned within the bore, the illuminating occurring during a surgical procedure on the patient;
the first LED light source (175) and the first camera (162) being located in a first magnetic resonance (MR) compatible casing having at least two separate openings (160,170);
the first camera (162) being oriented to view the patient through one of the separate openings (160); and
the first LED light source (175) being oriented to illuminate at least a portion of the patient through another of the separate openings (170).

12. The method of claim 11, further comprising:
supplying power to both the first LED light source (175) and the first camera (162) while the MRI system (500;600) is taking magnetic resonance (MR) images of the patient.

13. The method of claim 11 or 12, further comprising receiving images from the first camera (162) while the MRI system (500;600) is taking MR images of the patient.

14. The method of any one of claims 11 to 13, further comprising:
illuminating a second portion of the patient with a second LED light source (175) coupled to a second camera (162) positioned within the bore (610);
wherein the illuminating of both the first and second portions occurs at the same time;
the second LED light source (175) and the second camera (162) being located in a second magnetic resonance (MR) compatible casing having at least two separate openings (160,170;
the second camera (162) being oriented to view the patient through one of the separate openings (160); and
the second LED light source (175) being oriented to illuminate at least a portion of the patient through another of the separate openings (170).

15. The method of claim 14, wherein the first and second portions overlap to at least some extent.

16. The method of claim 14 or 15, further comprising:
supplying power to the second LED light source (175) and the second camera (162) while the MRI system (500;600) is taking MR images of the patient.

17. The method of any one of claims 14 to 16, further comprising receiving images from the second camera (162) while the MRI system (500;600) is taking MR images of the patient.

18. The method of any one of claims 14 to 17, further comprising:
orienting the first and second cameras (162) at a distance from each other and from a site to be imaged such that the images the cameras capture can be displayed as a three-dimensional stereoscopic image of the site.

19. The method of any one of claims 11 to 18, wherein the MRI system is an open bore MRI system (500).

20. The method of any one of claims 11 to 18, wherein the MRI system is a closed bore MRI system (600).

## Patentansprüche

1. Kamerasystem (100; 200), umfassend:
ein erstes Magnetresonanz(MR)-kompatibles Gehäuses mit mindestens zwei separaten Öffnungen (160, 170);
eine erste Kamera (162), die derart ausgerichtet ist, dass sie eine Stelle durch eine der separaten Öffnungen (160) betrachtet; und
eine erste Leuchtdioden(LED)-Lichtquelle (175);
**dadurch gekennzeichnet, dass** die erste LED-Lichtquelle (175) derart ausgerichtet ist, dass sie mindestens einen Abschnitt der Stelle durch eine andere der separaten Öffnungen (170) beleuchtet.

2. Kamerasystem nach Anspruch 1, ferner umfassend:
ein erstes abgeschirmtes Kabel, das Energie an die erste LED-Lichtquelle (175) liefert; und
erste abgeschirmte Video- und Netzkabel, die mit der ersten Kamera (162) gekoppelt sind.

3. Kamerasystem nach Anspruch 1 oder 2, wobei das Kamerasystem einen ersten oberen Abschnitt (110), der die erste Kamera und die erste LED-Lichtquelle aufweist, und eine erste Basis (120) aufweist, an welcher der erste obere Abschnitt (110) schwenkbar befestigt ist.

4. Kamerasystem nach Anspruch 3, wobei die Ausrichtung des ersten oberen Abschnitts (110) nach Entriegeln einer Steuerung (130), die mit der ersten Basis (120) gekoppelt ist, manuell eingestellt werden kann.

5. Kamerasystem nach einem der Ansprüche 1 bis 4, ferner umfassend:
ein zweites MR-kompatibles Gehäuse mit mindestens zwei separaten Öffnungen (160, 170);
eine zweite Kamera (162), die derart ausgerichtet ist, dass sie eine zweite Stelle durch eine der separaten Öffnungen (160) des zweiten Gehäuses betrachtet; und
eine zweite Leuchtdioden-Lichtquelle (175), die derart ausgerichtet ist, dass sie mindestens einen Abschnitt der zweiten Stelle durch eine andere der separaten Öffnungen (170) des zweiten Gehäuses beleuchtet.

6. Kamerasystem nach Anspruch 5, ferner umfassend:
ein zweites abgeschirmtes Kabel, das Strom an die zweite LED-Lichtquelle (175) liefert; und
zweite abgeschirmte Video- und Netzkabel, die mit der zweiten Kamera (162) gekoppelt sind.

7. Kamerasystem nach Anspruch 5 oder 6, wobei das Kamerasystem einen zweiten oberen Abschnitt (110), der die zweite Kamera (162) und die zweite LED-Lichtquelle (175) aufweist, und eine zweite Basis (120) aufweist, an welcher der zweite obere Abschnitt (110) schwenkbar befestigt ist.

8. Kamerasystem nach Anspruch 7, wobei die Ausrichtung des zweiten oberen Abschnitts (110) nach Entriegeln einer Steuerung (130), die mit der zweiten Basis (120) gekoppelt ist, manuell eingestellt werden kann.

9. Kamerasystem nach einem der Ansprüche 5 bis 8 zur Verwendung mit einem Manipulator (400), der dazu konfiguriert ist, an einem Verlängerungselement (300) gesichert zu werden, das mit einem Operationstisch (320) gekoppelt werden kann, auf dem ein Patient für einen stereotaktischen Eingriff angeordnet werden kann, wobei der Manipulator (400) dazu ausgelegt ist, ein chirurgisches Instrument zu bewegen, das sich in dem Tunnel (610) eines MRT-Magneten (500; 600) befindet, wobei das erste und das zweite Kamerasystem (100, 200) jeweils dazu konfiguriert sind, mit dem Verlängerungselement (300) gekoppelt zu werden.

10. Kamerasystem nach Anspruch 9, wobei das erste und das zweite Kamerasystem (100, 200) jeweils eine Lochblende (166) aufweisen.

11. Verfahren, umfassend:
Beleuchten eines ersten Abschnitts eines Patienten, der sich mindestens teilweise in dem Tunnel (610) eines Magneten eines Magnetresonanztomografie(MRT)-Systems (500; 600) befindet, mit einer ersten Leuchtdioden(LED)-Lichtquelle (175), die mit einer ersten Kamera (162) gekoppelt ist, die in dem Tunnel angeordnet ist, wobei das Beleuchten während eines chirurgischen Eingriffs an dem Patienten stattfindet;
wobei die erste LED-Lichtquelle (175) und die erste Kamera (162) in einem ersten Magnetresonanz(MR)-kompatiblen Gehäuse mit mindestens zwei separaten Öffnungen (160, 170) angeordnet sind;
die erste Kamera (162) derart ausgerichtet ist, dass sie den Patienten durch eine der separaten Öffnungen (160) betrachtet; und
die erste LED-Lichtquelle (175) derart ausgerichtet ist, dass sie mindestens einen Abschnitt des Patienten durch eine andere der separaten Öffnungen (170) beleuchtet.

12. Verfahren nach Anspruch 11, ferner umfassend:
Zuführen von Energie sowohl der ersten LED-Lichtquelle (175) als auch der ersten Kamera (162), während das MRT-System (500; 600) Magnetresonanz(MR)-Bilder von dem Patienten aufnimmt.

13. Verfahren nach Anspruch 11 oder 12, ferner umfassend das Empfangen von Bildern von der ersten Kamera (162), während das MRT-System (500; 600) MR-Bilder von dem Patienten aufnimmt.

14. Verfahren nach einem der Ansprüche 11 bis 13, ferner umfassend:
Beleuchten eines zweiten Abschnitts des Patienten mit einer zweiten LED-Lichtquelle (175), die mit einer zweiten Kamera (162) gekoppelt ist, die in dem Tunnel (610) angeordnet ist;
wobei das Beleuchten sowohl des ersten als auch des zweiten Abschnitts gleichzeitig stattfindet;
wobei die zweite LED-Lichtquelle (175) und die zweite Kamera (162) in einem zweiten Magnetresonanz(MR)-kompatiblen Gehäuse mit mindestens zwei separaten Öffnungen (160, 170) angeordnet sind;
die zweite Kamera (162) derart ausgerichtet ist, dass sie den Patienten durch eine der separaten Öffnungen (160) betrachtet; und
die zweite LED-Lichtquelle (175) derart ausgerichtet ist, dass sie mindestens einen Abschnitt des Patienten durch eine andere der separaten Öffnungen (170) beleuchtet.

15. Verfahren nach Anspruch 14, wobei sich der erste und der zweite Abschnitt mindestens in gewissem Maße überlappen.

16. Verfahren nach Anspruch 14 oder 15, ferner umfassend:
Zuführen von Energie sowohl der zweiten LED-Lichtquelle (175) als auch der zweiten Kamera (162), während das MRT-System (500; 600) MR-Bilder von dem Patienten aufnimmt.

17. Verfahren nach Anspruch 14 bis 16, ferner umfassend das Empfangen von Bildern von der zweiten Kamera (162), während das MRT-System (500; 600) MR-Bilder von dem Patienten aufnimmt.

18. Verfahren nach einem der Ansprüche 14 bis 17, ferner umfassend:
Ausrichten der ersten und der zweiten Kamera (162) in einem bestimmten Abstand voneinander und von einer Stelle, die abgebildet werden soll, sodass die Bilder, die von den Kameras erfasst werden, als ein dreidimensionales Bild der Stelle angezeigt werden können.

19. Verfahren nach einem der Ansprüche 11 bis 18, wobei das MRT-System ein MRT-System (500) mit offenem Tunnel ist.

20. Verfahren nach einem der Ansprüche 11 bis 18, wobei das MRT-System ein MRT-System (600) mit geschlossenem Tunnel ist.

## Revendications

1. Système de caméra (100 ; 200) comprenant :
un premier carter compatible avec la résonance magnétique (RM) comportant au moins deux ouvertures séparées (160, 170) ;
une première caméra (162) orientée pour visualiser un site à travers l'une des ouvertures séparées (160) ; et
une première source lumineuse (175) à diode électroluminescente (DEL) ;
**caractérisé en ce que** la source lumineuse (175) à DEL est orientée pour éclairer au moins une partie du site à travers une autre des ouvertures séparées (170).

2. Système de caméra selon la revendication 1, comprenant en outre :
un premier câble blindé qui conduit de l'électricité jusqu'à la première source lumineuse (175) à DEL ; et
des câbles vidéo et électriques blindés couplés à la première caméra (162).

3. Système de caméra selon la revendication 1 ou 2, dans lequel le système de caméra comprend une première partie supérieure (110) qui comprend la première caméra et la première source lumineuse à DEL, et une première base (120) à laquelle la première partie supérieure (110) est montée à pivot.

4. Système de caméra selon la revendication 3, dans lequel l'orientation de la première partie supérieure (110) peut être ajustée manuellement après déverrouillage d'un régulateur (130) couplé à la première base (120).

5. Système de caméra selon l'une quelconque des revendications 1 à 4, comprenant en outre :
un deuxième carter compatible avec la RM comportant au moins deux ouvertures séparées (160, 170) ;
une deuxième caméra (162) orientée pour visualiser un deuxième site à travers l'une des ouvertures séparées (160) du deuxième carter ; et
une deuxième source lumineuse (175) à diode électroluminescente orientée pour éclairer au moins une partie du deuxième site à travers une autre des ouvertures séparées (170) du deuxième carter.

6. Système de caméra selon la revendication 5, comprenant en outre :
un deuxième câble blindé qui conduit de l'électricité jusqu'à la deuxième source lumineuse (175) à DEL ; et
de deuxièmes câbles vidéo et électrique blindés, couplés à la deuxième caméra (162).

7. Système de caméra selon la revendication 5 ou 6, ce système de caméra comprenant une deuxième partie supérieure (110) contenant la deuxième caméra (162) et la deuxième source lumineuse (175) à DEL, et une deuxième base (120) à laquelle la deuxième partie supérieure (110) est montée à pivot.

8. Système de caméra selon la revendication 7, dans lequel l'orientation de la deuxième partie supérieure (110) peut être ajustée manuellement après déverrouillage d'un régulateur (130) couplée à la deuxième base (120).

9. Système de caméra selon l'une quelconque des revendications 5 à 8, utilisé avec un manipulateur (400) configuré pour être fixé à un élément d'extension (300) qui peut être couplé à une table d'opération (320) sur laquelle un patient peut être positionné pour une procédure stéréotactique, le manipulateur (400) étant opérable pour déplacer un instrument chirurgical situé dans le trou (610) d'un aimant d'IRM (500 ; 600), les premier et deuxième systèmes à caméra (100, 200) étant chacun configuré pour être couplé à l'élément d'extension (300).

10. Système de caméra selon la revendication 9, dans lequel les premier et deuxième systèmes de caméra (100, 200) comprennent chacun une lentille sténopéique (166).

11. Procédé comprenant :
l'éclairage d'une première partie d'un patient situé au moins en partie dans le trou (610) d'un aimant d'un système (500 ; 600) d'imagerie par résonance magnétique (IRM) avec une première source lumineuse (175) à diode électroluminescente (DEL) couplée à une première caméra (162) située dans le trou, l'éclairage ayant lieu pendant une procédure chirurgicale sur le patient ;
la première source lumineuse (175) à DEL et la première caméra (162) étant situées dans un premier carter compatible avec la résonance magnétique (RM) comptant au moins deux ouvertures séparées (160, 170) ;
la première caméra (162) étant orientée pour visualiser le patient par une des ouvertures séparées (160) ; et
la première source lumineuse (175) à DEL étant orientée pour éclairer au moins une partie du patient à travers une autre des ouvertures séparées (170).

12. Procédé selon la revendication 11, comprenant en outre :
l'alimentation de la première source lumineuse (175) à DEL et de la première caméra (162) tandis que le système à IRM (500 ; 600) prend des images par résonance magnétique (RM) du patient.

13. Procédé selon la revendication 11 ou 12, comprenant en outre la réception d'images à partir de la première caméra (162) alors que le système d'IRM (500 ; 600) prend des images de RM du patient.

14. Procédé selon l'une quelconque des revendications 11 à 13, comprenant en outre :
l'éclairage d'une deuxième partie du patient avec une deuxième source lumineuse (175) à DEL couplée à une deuxième caméra (162) positionnée dans le trou (610) ;
l'éclairage des première et deuxième parties ayant lieu au même moment ;
la deuxième source lumineuse (175) à DEL et la deuxième caméra (162) étant situées dans un deuxième carter compatible avec la résonance magnétique (RM) comptant au moins deux ouvertures séparées (160, 170) ;
la deuxième caméra (162) étant orientée pour visualiser le patient à travers une des ouvertures séparées (160) ; et
la deuxième source lumineuse (175) à DEL étant orientée pour éclairer au moins une partie du patient à travers une autre des ouvertures séparées (170).

15. Procédé selon la revendication 14, où les première et deuxième parties se chevauchent au moins dans une certaine mesure.

16. Procédé selon la revendication 14 ou 15, comprenant en outre :
l'alimentation électrique de la deuxième source lumineuse (175) à LED et de la deuxième caméra (162) pendant que le système d'IRM (500 ; 600) prend des images de RM du patient.

17. Procédé selon l'une quelconque des revendications 14 à 16, comprenant en outre la réception d'images depuis la deuxième caméra (162) tandis que le système d'IRM (500 ; 600) prend des images par RM du patient.

18. Procédé selon l'une quelconque des revendications 14 à 17, comprenant en outre :
l'orientation des première et deuxième caméras (162) à une certaine distance l'une de l'autre, et à partir d'un site à traiter par imagerie, de sorte que les images que les caméras prennent peuvent s'afficher sous la forme d'image stéréoscopique tridimensionnelle du site.

19. Procédé selon l'une quelconque des revendications 11 à 18, dans lequel le système d'IRM est un système à IRM à trou ouvert (500).

20. Procédé selon l'une quelconque des revendications 11 à 18, dans lequel le système d'IRM est un système à IRM à trou fermé (600).
